# EUROPÄISCHE PATENTSCHRIFT

(11) **EP 1 753 483 B1**
(45) Veröffentlichungstag und Bekanntmachung des Hinweises auf die Patenterteilung: **06.07.2011**
(21) Anmeldenummer: 05747274.8
(22) Anmeldetag: 07.06.2005
(51) Int. Cl.: A61M 1/10, A61M 25/10

(54) **VORRICHTUNG ZUR INTERMITTIERENDEN OKKLUSION DES KORONARSINUS**
DEVICE FOR THE INTERMITTENT OCCLUSION OF THE CORONARY SINUS
SYSTEME D'OCCLUSION INTERMITTENTE DU SINUS CORONAIRE

(30) Priorität: 08.06.2004 AT 9932004
(43) Veröffentlichungstag der Anmeldung: 21.02.2007
(73) Patentinhaber: MIRACOR Medical Systems GmbH, 1090 Wien (AT)
(72) Erfinder: Mohl, Werner, 2571 Altenmarkt/Thennenberg (AT)
(74) Vertreter: Haffner, Thomas M.
(86) Internationale Anmeldenummer: PCT/AT2005/000204
(87) Internationale Veröffentlichungsnummer: WO 2005/120602

(56) Entgegenhaltungen:
- WO-A-03/008018
- US-A- 4 934 996

## Beschreibung

Die Erfindung betrifft eine Vorrichtung für die intermittierende Okklusion des Koronarsinus umfassend eine Okklusions-Einrichtung, eine Druckmesseinrichtung zur kontinuierlichen Messung des Flüssigkeitsdrucks im okkludierten koronarsinus, einen Speicher für den Flüssigkeitsdruckverlauf als Funktion der Zeit und eine Auswerteschaltung zur Ermittlung des jeweils bei einem Herzschlag erfolgenden Druckanstiegs und/oder Druckabfalls je Zeiteinheit.

Eine derartige Vorrichtung ist der WO 03/008018 A zu entnehmen.

Arterielles Blut, das den Herzmuskel versorgt, kann durch gesundes Herzgewebe hindurchtreten und es ernähren, hat jedoch Schwierigkeiten das ischämische Gewebe zu erreichen. Dadurch wird die Versorgung des ischämischen Gewebes mit Nährstoffen sowie das Abführen von Abbauprodukten des Metabolismus von dem ischämischen Gewebe behindert.

In diesem Zusammenhang ist bereits vorgeschlagen worden das ischämische Gewebe durch retrograde Perfusion mit Blut zu versorgen. Dabei wird versucht das Blut in Gegenrichtung von dem Koronarsinus zurück durch das koronare Venensystem fließen zu lassen, in dem man aus einer anderen Quelle Blut in den Koronarsinus einspeist, entweder durch permanente Verbindung einer Arterie mit dem Koronarsinus oder durch temporäres Einsetzen eines Katheters in den Sinus, der mit von einer entfernten Arterie entnommenem und mit Hilfe einer sich außerhalb des Körpers des Patienten befindlichen Blutpumpe beförderten Blut versorgt wird.

Bei einer anderen vorgeschlagenen Technik für die Retroperfusion wird ein am Ende eines Katheters festgelegter aufblasbarer Ballon verwendet, um den Koronarsinus intermittierend zu okkludieren. Der Blutdruck im Koronareinus steigt während der okklusion bei jedem Herzschlag an, sodass Blut, das durch das gesunde Gewebe des Herzmuskels in den Koronarsinus gelangt, in das ischämische Gewebe zurückgeschwemmt wird. Bei einer solchen intermittierenden koronarsinusokklusion wird das Ballonende des Katheters perkutan oder durch eine Operation eingesetzt. Das andere Ende des Katheters wird mit Gas oder Flüssigkeit durch eine Pumpe versorgt, die ein zyklisches Aufblasen und Zusammenfallen des Ballons bewirkt.

Ein typisches Anwendungsgebiet für die Retroinfusion von Blut in Koronarvenen mittels intermittierender Koronarsinusokklusion betrifft die Myokardprotektion während eines kurzfristigen Koronararterienverschlusses im Rahmen eines kardiologischen Eingriffes. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauer-stoffhaltigem Blut statt, wobei sich bereits bei Dilatationen von länger als 30 Sekunden Dauer funktionelle Veränderungen im Ischämiegebiet des Myokards feststellen lassen. Entsprechende Probleme der Ischämieprotektion des Myokards stellen sich auch bei anderen Eingriffen zur Koronarvaskularisierung wie z.B. bei Atherektomie, Koronarendoprothesen und Laseranwendungen.

Eine Vorrichtung zur Retroinfusion von Koronarvenen ist beispielsweise aus der US 4,934,996 bekanntgeworden, mit welcher eine druckgesteuerte intermittierende Koronarsinusokklusion vorgenommen werden kann. Die Vorrichtung umfasst eine Einrichtung zum okkludieren des Sinus wie z.B. einen aufblasbaren Ballonkatheter, eine Druckmesseinrichtung zum Messen des Flüssigkeitadruckes innerhalb des Koronarsinus und ein Steuergerät, das Auslösesignale für die Okklusions-Einrichtung erzeugt, um eine Okklusion auszulösen oder aufzuheben. Das Steuergerät ist hiebei derart ausgelegt, dass im Koronarsinus das Druckmaximum während jedes Herzschlages gemessen, ein Plateauwert der Druckmaxima aufeinanderfolgender Herzschläge rechnerisch abgeschätzt und die Okklusion des Koronarsinus auf Basis des Plateauwertes der Druckmaxima aufgehoben wird.

Das Okkludieren des Koronarsinus bewirkt einen Druckanstieg und in der Folge eine Retroperfusion von Blut über die entsprechende Vene in die nutritiven Kapillaren des Ischämiegebietes, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden. Bei dem verfahren gemäß der US 4,934,996 wird somit aufgrund der Messung des Druckmaximums im Koronarsinus während jedes Herzschlages rechnerisch eine systolische Druckkurve abgeschätzt, wobei die intermittierende Okklusion in Abhängigkeit von dem Plateauwert der systolischen Druckkurve gesteuert wird. Der Verlauf der abgeschätzten systolischen Druckkurve lässt auch einen Rückschluss auf die Leistungsfähigkeit des Herzens zu, wobei beispielsweise die Steigung der Kurve die Kontraktibilität des Herzens wiedergibt.

Die Bestimmung des Zeitpunkts der Aufhebung der Okklusion anhand von Plateauwerten der systolischen Druckkurve gemäß der US 4,934,996 ist jedoch unter gewissen umständen nicht mit einer hinreichenden Genauigkeit möglich.

Die vorliegende Erfindung zielt nun darauf ab, eine vorrichtung für die intermittierende Okklusion des Koronarsinus vorzuschlagen, bei welchen Kenngrößen zur Steuerung der Okklusion herangezogen werden, welche das Bestimmen eines präzisen zeitpunktes erlauben, zu welchem die Okklusion des Koronarsinus aufgehoben oder ausgelöst wird. Gleichzeitig soll sichergestellt sein, dass der Zeitpunkt der Aufhebung oder des Auslösens der Okklusion hinsichtlich der Optimierung der therapeutischen und/oder diagnostischen Wirkung ausgewählt wird, wobei eine Schädigung des Herzmuskels verhindert werden soll.

Zur Lösung dieser Aufgabe ist die erfindungsgemäße Vorrichtung dadurch gekennzeichnet, dass die Auswerteschaltung mit der Okklusions-Einrichtung zum Auslösen und/oder Aufheben der Okklusion des Koronarsinus in Abhängigkeit von dem genannten Druckanstieg bzw. -abfall je zeiteinheit zusammenwirkt. Der Druckanstieg bzw. Druckabfall je Zeiteinheit gibt hierbei jeweils die innerhalb eines Herzschlages zu beobachtende positive bzw. negative Steigung der Flüssigkeitsdruckkurve wieder und errechnet sich ausgehend von dem Flüssigkeitsdruckverlauf als Funktion der Zeit und dem aus dieser Funktion innerhalb eines Zeitintervalls (Δt) ablesbaren Druckanstieg bzw. Druckabfall (Ap) wie folgt: Δp/Δt. Diese Kenngröße erlaubt es dem behandelnden Arzt sowohl während der Okklusion des Koronarsinus (Okklusionsphase) als auch nach der Aufhebung der Okklusion (Releasephase) Rückschlüsse auf die Kontraktibilität des Herzens und auf die Verhältnisse innerhalb des Koronarsinus zu ziehen, um daraus die optimalen Okklusionszeiten abzuleiten. Überraschender Weise ist es hierfür nicht nötig den innerhalb des Koronarsinus herrschenden Druck in absoluten Zahlen zu kennen, sondern es reicht erfindungsgemäß aus, Druckdifferenzen, nämlich den jeweils innerhalb eines Herzschlages erfolgenden Druckanstieg oder Druckabfall je Zeiteinheit, auszuwerten.

Der Druckverlauf im okkludierten Koronarsinus verläuft in der Regel derart, dass bei jedem Herzschlag die Geschwindigkeit des Druckanstiegs größer wird und somit immer höhere systolische Druckspitzen auftreten, bis eine sogenannte "Plateauphase" erreicht wird, wo zwar die Geschwindigkeit des Druckanstiegs bei aufeinanderfolgenden Herzschlägen weiter zunimmt, der systolische Druck aber im wesentlichen bereits einen Plateauwert erreicht hat und im wesentlichen gleich bleibt. Erst wenn die Kontraktibilität des Herzens durch arterielle Minderperfusion (koronare widerstandserhöhung) in dieser Plateauphase zurückgeht, nimmt die Geschwindigkeit des Druckanstiegs wieder ab, wobei der systolische Druck im Wesentlichen weiter auf dem Plateauwert bleibt. Wenn nun erfindungsgemäß abweichend von dem stand der Technik für die Bestimmung des optimalen zeitpunkts der Aufhebung der Okklusion nicht absolute Druckwerte, wie beispielsweise der Plateauwert der systolischen Druckspitzen, sondern die Geschwindigkeit des Druckanstiegs herangezogen wird, kann die Okklusion länger beibehalten werden ohne dass das Herz beeinträchtigt wird. Es kann nämlich die Okklusion beispielsweise erst zu dem Zeitpunkt aufgehoben werden, zu welchem die Geschwindigkeit des Druckanstiegs während aufeinanderfolgender Herzschläge ein Maximum erreicht hat, oder zu einem zeitpunkt, zu welchem ein vorbestimmter Prozentsatz des errechneten oder abgeschätzten Maximums der Geschwindigkeit des Druckanstiegs erreicht ist, wobei dieser Zeitpunkt nach dem Erreichen des systolischen Plateauwerts liegt. Der Umstand, dass die Okklusion länger beibehalten werden kann ohne das Herz oder die Herzgefäße zu überlasten oder zu schädigen, führt in vorteilhafter Weise dazu, dass es durch die möglichst lang andauernde Druckerhöhung vermehrt zur Freisetzung von gefäßbildenden Genen (VEGF-Gene, den vaskulären endothelialen Wachstumsfaktor kodierende Gene) kommt, sodass auch die Regeneration der Gefäße begünstigt wird.

Die Steuerung der Aufhebung oder Auslösung der Okklusion in Abhängigkeit von dem Druckanstieg bzw. Druckabfall je Zeiteinheit kann auf verschiedenste weise erfolgen. Beispielsweise könnte die Okklusion aufgehoben werden, sobald ein vorbestimmter oberer Grenzwert für die Kenngröße Δp/Δt überschritten wird, oder umgekehrt die Okklusion ausgelöst werden, wenn die Kenngröße Δp/Δt einen unteren Grenzwert unterschreitet. Gemäß einer bevorzugten Ausbildung ist dabei vorgesehen, dass die Auswerteschaltung zur Ermittlung des jeweils bei einem Herzschlag erfolgenden Druckanstiegs je Zeiteinheit ausgebildet ist und mit der Okklusions-Einrichtung derart zusammenwirkt, dass die Okklusion des Ko-ronarsinus aufgehoben wird, nachdem der Druckanstieg je Zeiteinheit aufeinanderfolgender Herzschläge ein Maximum erreicht hat. Dadurch erfolgt eine präzise Ermittlung der optimalen Dauer der Okklusionszeit. In analoger Weise kann für die Dauer der Releasephase derart vorgegangen werden, dass der bei einem Herzschlag erfolgende Druckabfall je Zeiteinheit ermittelt wird und die Okklusion ausgelöst wird, nachdem der Druckabfall je Zeiteinheit aufeinanderfolgender Herzschläge ein Minimum erreicht hat.

Eine weitere Verfeinerung des Verfahrens, welche sich mit Hilfe mathematischer Algorithmen besonders leicht umsetzen lässt, gelingt für die Okklusionsphase dadurch, dass die Auswerteschaltung zur Ermittlung der ersten Ableitung des Flüssigkeitsdrucks nach der zeit und des jeweils innerhalb eines Herzschlags auftretenden lokalen Maximums der ersten Ableitung ausgebildet ist und mit der Okklusions-Einrichtung derart ausammenwirkt, dass die Okklusion des Koronarsinus aufgehoben wird, nachdem die lokalen Maxima aufeinanderfolgender Herzschläge ein Maximum erreicht haben. In analoger weise ergibt sich hierbei für die Releasephase mit Vorteil eine Ausbildung, bei welcher die Auswerteschaltung zur Ermittlung der ersten Ableitung des Flüssigkeitsdrucks nach der Zeit und des jeweils innerhalb eines Herzschlags auftretenden lokalen Minimums der ersten Ableitung ausgebildet ist und mit der Okklusions-Einrichtung derart zusammenwirkt, dass die Okklusion des Koronarsinus ausgelöst wird, nachdem die lokalen Minima aufeinanderfolgender Herzschläge ein Minimum erreicht haben.

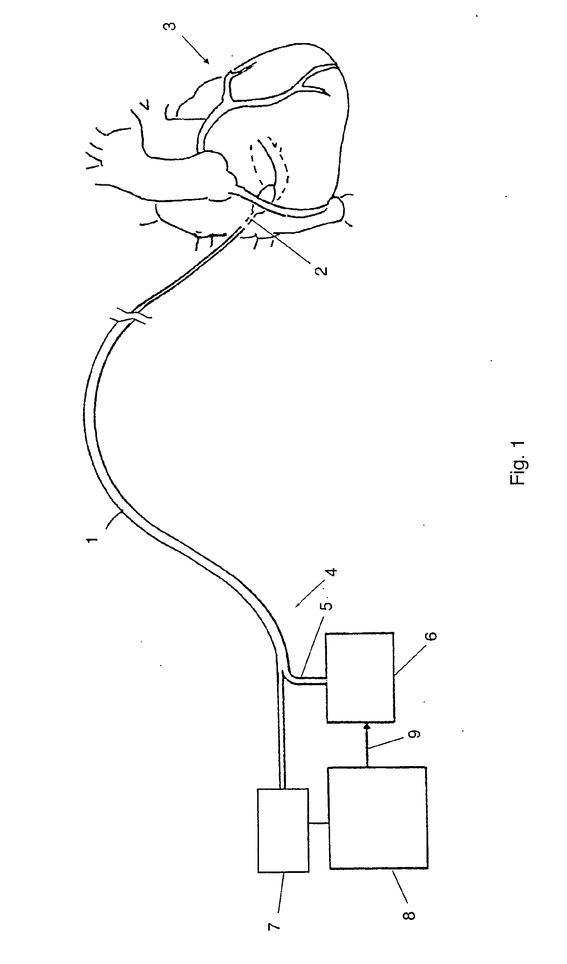

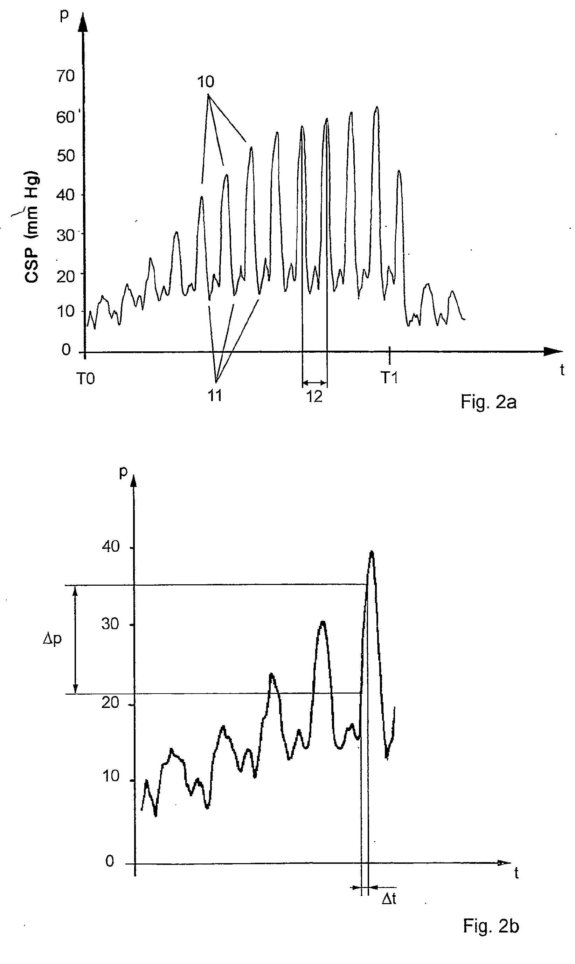

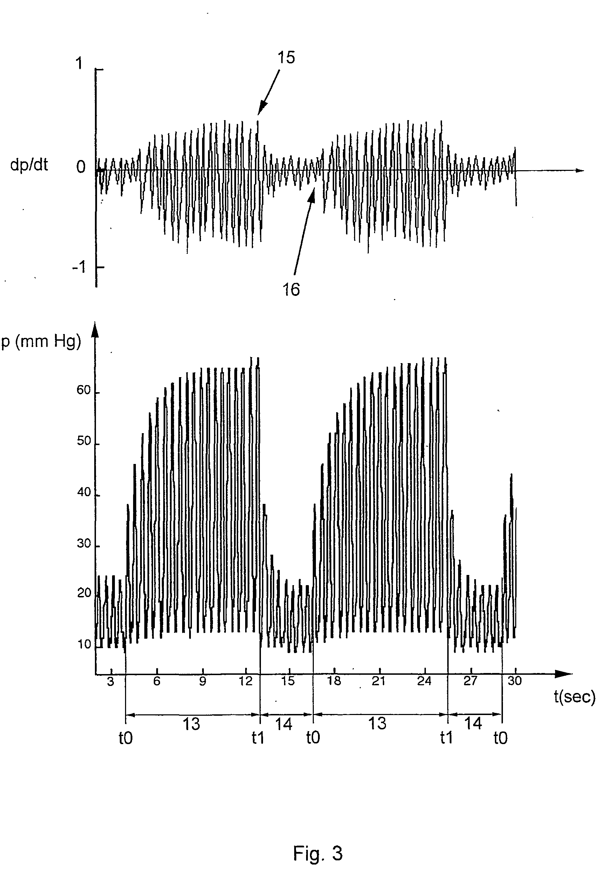

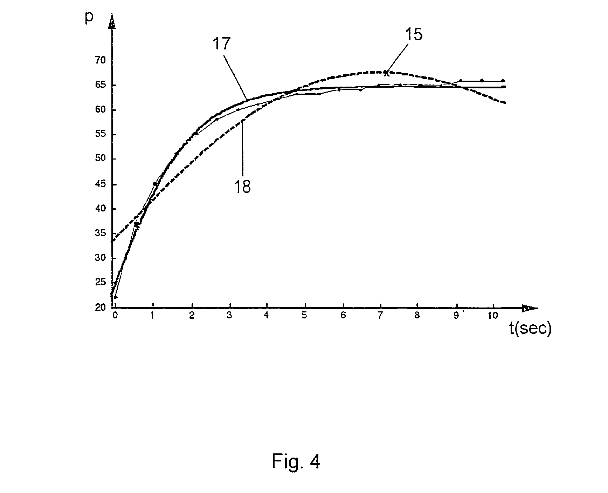

Die Erfindung wird nun nachfolgend anhand eines in der Zeichnung dargestellten Ausführungsbeiepiels näher erläutert. In dieser zeigen Fig.1 eine Diagrammansicht eines Herzens mit einer Vorrichtung zum intermittierenden Okkludieren des Koronarsinus, Fig.2 eine graphische Darstellung des Koronareinuadruckverlaufes, Fig.3 eine Gegenüberstellung des Druckverlaufes und der Ableitung des Druckverlaufes nach der Zeit und Fig.4 die Hüllkurven der Druckverläufe gemäß Fig.3.

In Fig. 1 ist schematisch die Vorrichtung zum intermittierenden Okkludieren des Koronarsinus dargestellt, wobei ein Multilumen-Katheter 1, dessen distales Ende 2 in den Koronarsinus des Herzens 3 über das Atrium eingesetzt wird, ersichtlich ist. Das proximale Ende 4 des Katheters 1 hat ein Ballonaufblaslumen 5, das mit einer Pumpe 6 verbunden ist. Der am distalen Ende 2 des Katheters 1 herrschende Druck wird von einer Druckmesseinrichtung 7 erfasst, wobei die Druckmesseinrichtung auch einen Speicher für die ermittelten Messwerte aufweist. Die jeweiligen Druckmesswerte werden einem Steuergerät mit einer Auswertschaltung 8 zugeführt, um Steuersignale über die Leitung 9 zum Starten und Anhalten der Pumpe 6 zu liefern.

In Fig. 2a ist der von der Messeinrichtung 7 erfasste Druckverlauf dargestellt, wobei der Beginn der Okklusion mit T0 und das Ende der Okklusion mit T1 dargestellt ist. Es ist eine Reihe von systolischen Druckspitzen 10 und eine Reihe von diastolischen Tälern 11 ersichtlich. Die Pulsperiode 12 des Herzschlages wird durch die Zeit zwischen aufeinanderfolgenden Spitzen oder aufeinanderfolgenden Tälern darge-stellt. Während jedes Herzschlags innerhalb der Okklusionsphase 13 wird die Geschwindigkeit des Druckanstieges durch Berechnung der Verhältnisses Δp/Δt ermittelt, wie dies schematisch in der vergrößerten Darstellung in Fig.2b dargestellt ist. Analoges erfolgt in der Releasephase 14 für die Geschwindigkeit des Druckabfalls. Durch eine Grenzwertberechnung ergibt sich für einen gegen 0 strebenden zeitintervall Δt die Darstellung gemäß Fig.3, in welcher der Kurve des Druckverlaufes die entsprechende Kurve der ersten Ableitung dp/dt des Druckverlaufes gegenüber gestellt ist. Es ist ersichtlich, dass die erste Ableitung an denjenigen Stellen, an welchen die Steigung der Druckkurve innerhalb jedes Herzschlages am größten ist, ein Maximum erreicht. Ebenso ist ersichtlich, dass die erste Ableitung an denjenigen Stellen, an denen die negative Steigung des Druckverlaufes innerhalb jedes Herzschlages am geringsten ist, ein Minimum erreicht. Da in der Folge lediglich die jeweils innerhalb eines Herzschlages erfolgenden Druckmaxima bzw. Druckminima sowie die innerhalb jedes Herzschlages zu beobachtenden Maxima und Minima der ersten Ableitung der Druckkurve von Bedeutung sind, sind in Fig.4 der Übersichtlichkeit halber lediglich die entsprechenden Hüllkurven dargestellt, welche die entsprechenden Maxima verbinden. Dabei ist in Fig.4 beispielhaft lediglich die Hüllkurve für die Phase der Okklusion dargestellt.

Während der Okklusion des Koronarsinus ist zu beobachten, dass die Geschwindigkeit des Druckanstieges bei aufeinanderfolgenden Herzschlägen immer größer wird, wie dies anhand der Hüllkurve 17 der ersten Ableitung abzulesen ist. Der optimale Zeitpunkt für das Aufheben der Okklusion ergibt sich an derjenigen Stelle 15, an welcher die Hüllkurve 17 ihr Maximum erreicht, d.h. an welcher die Geschwindigkeit des Druckanstieges aufeinanderfolgender Herzschläge am größten ist. Dieser Zeitpunkt lässt sich mathematisch am einfachsten dadurch bestimmen, dass die zweite Ableitung der Druckkurve gleich 0 gesetzt wird. Dabei ergibt sich, dass an der Stelle 15 die Hüllkurve 17 der ersten Ableitung der Druckkurve ein Maximum erreicht, wohingegen die Hüllkurve 18, welche die systolischen Druckspitzen verbindet, schon zuvor einen Plateauwert erreicht hat. In analoger Weise ergibt sich der optimale Zeitpunkt für das Auslösen der Okklusion die Stelle 16 (Fig.3).

Durch Aufheben der Okklusion an der Stelle 15 wird einerseits sichergestellt, dass die okklusion möglichst lange aufrechterhalten wird, sodass im okkludierten Koronarsinus möglichst lange eine Druckniveau herrscht, welches die Freisetzung von VEGF-Genen fördert. Andererseits werden schädliche Nebenwirkungen durch eine zu lange andauernde Okklusion vermieden, da die Okklusion rechtzeitig aufgehoben wird, sobald die Kontraktibilität des Herzens zurückgeht.

Zusammenfassend wird durch die vorliegende Erfindung ein einfach durchzuführendes Verfahren bzw. eine Vorrichtung, welche auf einem einfach zu verwirklichenden Algorithmus basiert, vorgeschlagen, wobei eine optimale und präzise Bestimmung der okklusionszeiten und der Releasezeiten gelingt.

## Patentansprüche

1. Vorrichtung zur intermittierenden Okklusion des Koro-narsinus umfassend eine Okklusions-Einrichtung, eine Druckmesseinrichtung (7) zur kontinuierlichen Messung des Flüssigkeitsdrucks im okkludierten Koronarsinus, einen Speicher für den Flüssigkeitsdruckverlauf als Funktion der zeit und eine Auswerteschaltung (8) zur Ermittlung des jeweils bei einem Herzschlag erfolgenden Druckanstiegs und/oder Druckabfalls je Zeiteinheit, **dadurch gekennzeichnet, dass** die Auswerteschaltung (8) mit der Okklusions-Einrichtung zum Auslösen und/oder Aufheben der Okklusion des Koronarsinus in Abhängigkeit von dem genannten Druckanstieg bzw. -abfall je zeiteinheit zusammenwirkt.

2. Vorrichtung nach Anspruch 1, **dadurch gekennzeichnet, dass** die Auswerteschaltung (8) zur Ermittlung des jeweils bei einem Herzschlag erfolgenden Druckanstiegs je Zeiteinheit ausgebildet ist und mit der Okklusions-Einrichtung derart zusammenwirkt, dass die Okklusion des Koronarsinus aufgehoben wird, nachdem der Druckanstieg je zeiteinheit aufeinanderfolgender Herzschläge ein Maximum erreicht hat.

3. Vorrichtung nach Anspruch 1 oder 2, **dadurch gekennzeichnet, dass** die Auswerteschaltung (8) zur Ermittlung des jeweils bei einem Herzschlag erfolgenden Druckabfalls je Zeiteinheit ausgebildet ist und mit der Okklusions-Einrichtung derart zusammenwirkt, dass die Okklusion des Koronarsinus ausgelöst wird, nachdem der Druckabfall je Zeiteinheit aufeinanderfolgender Herzschläge ein Minimum erreicht hat.

4. Vorrichtung nach Anspruch 1, 2 oder 3, **dadurch gekennzeichnet, dass** die Auswerteschaltung (8) zur Ermittlung der ersten Ableitung des Flüssigkeitsdrucks nach der Zeit und des jeweils innerhalb eines Herzschlags auftretenden lokalen Maximums der ersten Ableitung ausgebildet ist und mit der Okklusions-Einrichtung derart zusammenwirkt, dass die Okklusion des Koronarsinus aufgehoben wird, nachdem die lokalen Maxima aufeinanderfolgender Herzschläge ein Maximum erreicht haben.

5. Vorrichtung nach einem der Ansprüche 1 bis 4, **dadurch gekennzeichnet, dass** die Auswerteschaltung (8) zur Ermittlung der ersten Ableitung des Flüssigkeitsdrucks nach der Zeit und des jeweils innerhalb eines Herzschlags auftretenden lokalen Minimums der ersten Ableitung ausgebildet ist und mit der Okklusions-Einrichtung derart zusammenwirkt, dass die Okklusion des Koronarsinus ausgelöst wird, nachdem die lokalen Minima aufeinanderfolgender Herzschläge ein Minimum erreicht haben.

## Claims

1. A device for the intermittent occlusion of the coronary sinus including an occlusion device, a pressure measuring device (7) for continuously measuring the fluid pressure in the occluded coronary sinus, a memory for storing the fluid pressure curve as a function of time and an evaluation circuit (8) for the determination of the pressure increase and/or pressure decrease per time unit each occurring at a heart beat, **characterized in that** the evaluation circuit (8) cooperates with the occlusion device to trigger and/or release the occlusion of the coronary sinus depending on said pressure increase and/or pressure decrease per time unit.

2. A device according to claim 1, **characterized in that** the evaluation circuit (8) is configured to determine the pressure increase per time unit each occurring at a heart beat, and cooperates with the occlusion device in a manner that the occlusion of the coronary sinus is released after the pressure increase per time unit of consecutive heart beats has reached a maximum.

3. A device according to claim 1 or 2, **characterized in that** the evaluation circuit (8) is configured to determine the pressure decrease per time unit each occurring at a heart beat, and cooperates with the occlusion device in a manner that the occlusion of the coronary sinus is triggered after the pressure decrease per time unit of consecutive heart beats has reached a minimum.

4. A device according to claim 1, 2 or 3, **characterized in that** the evaluation circuit (8) is configured to determine the first derivative of the fluid pressure with respect to time as well as the local maximum of the first derivative each occurring within a heart beat, and cooperates with the occlusion device in a manner that the occlusion of the coronary sinus is released after the local maxima of consecutive heart beats have reached a maximum.

5. A device according to any one of claims 1 to 4, **characterized in that** the evaluation circuit (8) is configured to determine the first derivative of the fluid pressure with respect to time as well as the local minimum of the first derivative each occurring within a heart beat, and cooperates with the occlusion device in a manner that the occlusion of the coronary sinus is triggered after the local minima of consecutive heart beats have reached a minimum.

## Revendications

1. Appareil pour l'occlusion intermittente du sinus coronaire, comprenant un dispositif d'occlusion, un dispositif de mesure de pression (7) pour la mesure continue de la pression de liquide dans le sinus coronaire occlus, une mémoire de courbe de pression de liquide en fonction du temps et un circuit d'évaluation (8) pour une détermination par unité de temps de l'élévation de pression et/ou de la diminution de pression survenant lors d'une pulsation cardiaque, **caractérisé en ce que** le circuit d'évaluation (8) coopère avec le dispositif d'occlusion pour le déclenchement et/ou l'annulation de l'occlusion du sinus coronaire en fonction de ladite élévation ou de ladite diminution de pression par unité de temps.

2. Appareil selon la revendication 1, **caractérisé en ce que** le circuit d'évaluation (8) est prévu pour la détermination par unité de temps de l'élévation de pression survenant lors d'une pulsation cardiaque et coopère avec le dispositif d'occlusion de manière à annuler l'occlusion du sinus coronaire lorsque l'élévation de pression par unité de temps de pulsations cardiaques consécutives atteint un maximum.

3. Appareil selon la revendication 1 ou la revendication 2, **caractérisé en ce que** le circuit d'évaluation (8) est prévu pour la détermination par unité de temps de la diminution de pression survenant lors d'une pulsation cardiaque et coopère avec le dispositif d'occlusion de manière à déclencher l'occlusion du sinus coronaire lorsque la diminution de pression par unité de temps de pulsations cardiaques consécutives atteint un minimum.

4. Appareil selon la revendication 1, la revendication 2 ou la revendication 3, **caractérisé en ce que** le circuit d'évaluation (8) est prévu pour la détermination de la première dérivée de la pression de liquide par rapport au temps et du maximum local de la première dérivée survenant pendant une pulsation cardiaque, et coopère avec le dispositif d'occlusion de manière à annuler l'occlusion du sinus coronaire lorsque les maxima locaux de pulsations cardiaques consécutives atteignent un maximum.

5. Appareil selon l'une des revendications 1 à 4, **caractérisé en ce que** le circuit d'évaluation (8) est prévu pour la détermination de la première dérivée de la pression de liquide par rapport au temps et du minimum local de la première dérivée survenant pendant une pulsation cardiaque, et coopère avec le dispositif d'occlusion de manière à déclencher l'occlusion du sinus coronaire lorsque les minima locaux de pulsations cardiaques consécutives atteignent un minimum.
